# EUROPEAN PATENT APPLICATION

(11) **EP 2 050 461 A1**
(43) Date of publication of application: **22.04.2009**
(21) Application number: 07118922.9
(22) Date of filing: 19.10.2007
(51) Int. Cl.: A61K 38/40, C07K 14/79

(54) **Peptides based on the sequence of human lactoferrin and their use**

(71) Applicant: PharmaSurgics in Sweden AB, 413 46 Göteborg (SE)
(72) Inventor: Mattsby-Baltzer, Inger, 412 70, GÖTEBORG (SE); Hansson, Lars, 411 23, GÖTEBORG (SE); Baltzer, Lars, 753 10, UPPSALA (SE); Dolphin, Gunnar, 730 00, CHAMBÉRY (FR)
(74) Representative: Andersson, Inga-Lill

(57) **Abstract**

The present invention relates to new peptides designed based on the sequence of human lactoferrin and to use thereof, in particular for treatment and/or prevention of infections, inflammations, tumours, pain, wounds and/or scars.

SEQUENCE LISTING

<110>  PHARMASURGICS IN SWEDEN AB

<120>  NEW SYNTHETIC PEPTIDES AND THEIR USE

<130>

<140>

<160>  4

<170>  PatentIn version 3.3

<210>  1
<211>  12
<212>  PRT
<213>  Artificial

<220>
<223>  a fragment of human lactoferrin corresponding to aa 20-31

<400>  1

Cys Phe Gln Trp Gln Arg Asn Met Arg Lys Val Arg
1               5                   10

<210>  2
<211>  25
<212>  PRT
<213>  Artificial

<220>
<223>  a fragment of human lactoferrin corresponding to aa 16-40
       wherein one aa has been substituted

<400>  2

Glu Ala Thr Lys Cys Phe Gln Trp Lys Arg Asn Met Arg Lys Val Arg
1               5                   10                  15

Gly Pro Pro Val Ser Cys Ile Lys Arg
            20                  25

<210>  3
<211>  25
<212>  PRT
<213>  Artificial

<220>
<223>  a fragment of human lactoferrin corresponding to aa 16-40
       wherein two aa has been substituted

<400>  3

Glu Ala Thr Lys Cys Phe Gln Trp Lys Arg Ala Met Arg Lys Val Arg
1               5                   10                  15

Gly Pro Pro Val Ser Cys Ile Lys Arg
            20                  25

<210>  4
<211>  25
<212>  PRT

<213>  Artificial

<220>
<223>  a fragment of human lactoferrin corresponding to aa 16-40

<400>  4

Glu Ala Thr Lys Cys Phe Gln Trp Gln Arg Asn Met Arg Lys Val Arg
1               5                   10                  15

Gly Pro Pro Val Ser Cys Ile Lys Arg
            20                  25

## Description

### Field of the invention

The present invention relates to new peptides and to use thereof, in particular for treatment and/or prevention of infections, inflammations, tumours, pain, wounds and/or scars.

### Background art

Lactoferrin is a single chain metal binding glycoprotein with a molecular weight of 77 kd. It has been found that the structural domain of lactoferrin responsible for the bactericidal properties is a pepsin-cleaved fragment called lactoferricin (see e. g. Bellamy W., et al., Identification of the bactericidal domain of lactoferrin, Biochim. Biophys. Acta 1121: 130-136, 1992, and Bellamy W., et al., Antibacterial spectrum of lactoferricin B, a potent bactericidal peptide derived from the N-terminal region of bovine lactoferrin, J. Appl. Bact. 73: 472-479, 1992).

Lactoferrin receptors are found on many types of cells including monocytes and macrophages, lectin-stimulated human peripheral blood lymphocytes, brushborder cells, and tumour cell lines.

Several patent publications describe the possible use of lactoferrin for treatment of infections or inflammations. In WO 98/06425, e. g., it is disclosed that lactoferrin and lactoferricin can be used for treatment and prevention of infections, inflammations and tumours.

EP 629 347 describes an antimicrobial agent containing (A) lactoferrin hydrolysate and/or one or more of antimicrobial peptides derived from lactoferrins, and (B) one or more compounds selected from the group consisting of metal-chelating protein, tocopherol, cyclodextrin, glycerine-fatty acid ester, alcohol, EDTA or a salt thereof, ascorbic acid or a salt thereof, citric acid or a salt thereof, polyphosphoric acid or a salt thereof, chitosan, cysteine, and cholic acid as the effective components thereof. This antimicrobial agent is intended for treatment of products, and especially for safely treating e. g. food and medicines. The agent according to this publication is thus a new preservative. In the publication several peptide sequences are given and some of them resemble the peptides according to the invention, although there are several important differences described further below.

US 5,304,633 discloses antimicrobial peptides isolated from hydrolysates of human and bovine lactoferrin. Isolation of peptides corresponding to amino acids 12 to 47, and 17 to 41 of human lactoferrin are specifically disclosed.

JP 7145196 describes the preparation of antibiotic peptides by hydrolysis of lactoferrin. The preparation of a peptide corresponding to amino acids 17 to 41 of human lactoferrin is specifically described.

JP 8040925 discloses pharmaceutical compositions containing lactoferrin derived peptides and their use in the treatment of cornea damages, especially keratitis. Peptides corresponding to amino acids 17 to 41, 12 to 58, and 19 to 38, of human lactoferrin are specifically disclosed

JP 7274970 describes the recombinant production of antibacterial lactoferricin derived peptides, specifically a peptides corresponding to amino acids 18 to 42 of human lactoferrin is disclosed.

JP 8143468 describes lactoferrin derived peptides and their use as antiulcer drugs, a peptide corresponding to amino acids 19 to 33 of human lactoferrin is specifically disclosed.

WO 00/01730 describes peptides derived from human lactoferrin and their use for treatment of infections and inflammations.

EP 1 228 097 describes peptides derived from the immediate N-terminal end of human lactoferrin and their use as microbial agents.

EP 1151009 describes peptides comprising a sequence corresponding to amino acids 35 to 50 of human lactoferrin having antimicrobial and/or endotoxin neutralizing activity.

WO 2006/047744 describes immunomodulatory peptides derived from the N-terminal part of human lactoferrin comprising at least 33 amino acids and being substituted in both the N- and C-terminus with four positively charged amino acids.

### Summary of the invention

The object of the present invention is to provide new synthetic peptides which can be used for the same purposes as lactoferrin, lactoferricin or other lactoferrin derived peptides and which will have the same, or better, effects although having production, technical and/or biochemical advantages.

The aim of the studies leading to the present invention was to design new peptides which should essentially be as efficient as, or preferably more efficient, than human lactoferrin, human lactoferricin and other lactoferrin derived peptides in treatment and prevention of infections, inflammations, tumours, wounds, and scars.

It was found that peptides formed of the sequences constituted of at least amino acids 20-31 of human lactoferrin counted from the N-terminal end, where one or more amino acid has been substituted, have the desired properties.

It has been shown that humans in their brush border membrane have receptors which can bind to human lactoferrin (see e. g. Lonnerdal B., Lactoferrin receptors in intestinal brush border membranes, Adv. Exp. Med. Biol. 1994, 357: 171-175). It has also been shown that bovine lactoferrin does not bind to these receptors. A plausible mechanism for the uptake of these new peptides in the human body is that the peptides are taken up through binding to cellular receptors. However, the invention is in no way limited to this mechanism.

Thus, the present invention relates to new synthetic peptides and to functionally equivalent homologues or analogues thereof.

Furthermore, the invention relates to medicinal products and to food stuff, especially infant formula food, comprising said peptides.

The invention also relates to use of said peptides for the production of medicinal products for treatment and prevention of infections, inflammations and tumours.

The peptides according to the invention are fungicidal and bactericidal, and can thus be used for other applications when substances with such properties are desired. They may for example be used as preservatives.

The characterising features of the invention will be evident from the following description and the appended claims.

### Detailed description of the invention

Thus, the present invention relates to peptides designed based on the amino acid sequence of fragments of the protein human lactoferrin (hLF). The fragment of hLF that is used as a basis for the invention is constituted by the amino acids in positions 20-31 of hLF, the sequence of which is:

Cys-Phe-Gln-Trp-Gln-Arg-Asn-Met-Arg-Lys-Val-Arg (SEQ ID NO:1)

Thus, the present invention relates to peptides according to formula (I)

R1-Cys-Phe-X1-X2-X3-X4-X5-X6-X7-Lys-Val-Arg-R2 Formula (I)

wherein amino acid X1 is Gln or Ala, amino acid X2 is Trp or Leu, amino acid X3 is Gln, Ala, Orn, Nle or Lys, amino acid X4 is Arg, Ala or Lys, amino acid X5 is Asn, Ala, Orn or Nle, amino acid X6 is Met, Ala or Leu, amino acid X7 is Arg, Ala or Lys; with the proviso that simultaneously can not X1 be Gln, X2 be Trp, X3 be Gln, X4 be Arg, X5 be Asn, X6 be Met, and X7 be Arg.
R1 is either Lys or a peptide sequence selected from

| |
|---|
| Gly-Arg-Arg-Arg-Arg-Ser-Val-Gln-Trp-Cys-Ala-Val-Ser-Gln-Pro-Glu-Ala-Thr-Lys, |
| Arg-Arg-Arg-Arg-Ser-Val-Gln-Trp-Cys-Ala-Val-Ser-Gln-Pro-Glu-Ala-Thr-Lys, |
| Arg-Arg-Arg-Ser-Val-Gln-Trp-Cys-Ala-Val-Ser-Gln-Pro-Glu-Ala-Thr-Lys, |
| Arg-Arg-Ser-Val-Gln-Trp-Cys-Ala-Val-Ser-Gln-Pro-Glu-Ala-Thr-Lys, |
| Arg-Ser-Val-Gln-Trp-Cys-Ala-Val-Ser-Gln-Pro-Glu-Ala-Thr-Lys, |
| Ser-Val-Gln-Trp-Cys-Ala-Val-Ser-Gln-Pro-Glu-Ala-Thr-Lys, |
| Val-Gln-Trp-Cys-Ala-Val-Ser-Gln-Pro-Glu-Ala-Thr-Lys, |
| Gln-Trp-Cys-Ala-Val-Ser-Gln-Pro-Glu-Ala-Thr-Lys, |
| Trp-Cys-Ala-Val-Ser-Gln-Pro-Glu-Ala-Thr-Lys, |
| Cys-Ala-Val-Ser-Gln-Pro-Glu-Ala-Thr-Lys, |
| Ala-Val-Ser-Gln-Pro-Glu-Ala-Thr-Lys, |
| Val-Ser-Gln-Pro-Glu-Ala-Thr-Lys, |
| Ser-Gln-Pro-Glu-Ala-Thr-Lys, |
| Gln-Pro-Glu-Ala-Thr-Lys, |
| Pro-Glu-Ala-Thr-Lys, |
| Glu-Ala-Thr-Lys, |
| Ala-Thr-Lys, |
| Thr-Lys, |

R2 is either Gly or a peptide sequence selected from
Gly-Pro-Pro-Val-Ser-Cys-Ile-Lys-Arg,
Gly-Pro-Pro-Val-Ser-Cys-Ile-Lys,
Gly-Pro-Pro-Val-Ser-Cys-Ile,
Gly-Pro-Pro-Val-Ser-Cys,
Gly-Pro-Pro-Val-Ser,
Gly-Pro-Pro-Val,
Gly-Pro-Pro, and
Gly-Pro
In one preferred aspect of the invention amino acid X3 is Lys.
In one preferred aspect of the invention amino acid X5 is Ala.
In one preferred aspect of the invention R1 is Glu-Ala-Thr-Lys.
In one preferred aspect of the invention R2 is Gly-Pro-Pro-Val-Ser-Cys-Ile-Lys-Arg

When present, it may be advantageous to replace the amino acid Cys by an acetamidomethyl-cysteine in order to avoid that the peptide forms a disulphide bridge with another peptide comprising a cysteine.

According to one preferred aspect of the invention the carboxy terminal end of the peptide has been capped, i. e. the free COOH at the carboxy terminal end has been transformed into CONH₂.

According to another preferred aspect of the invention the amino terminal end of the peptide has been capped, i. e. the free NH₂ group at the amino terminal has been transformed into the amide CH₃CONH- (AcNH-).

According to yet another preferred aspect of the invention both the carboxy-terminal and the amino-terminal ends of the peptide have been capped.

The advantage of the capped versions is that N- and C-terminal amino acids of these peptides are neutral and uncharged and thus has changed electrostatic properties. Assuming that the receptors bind the corresponding sequences of human lactoferrin where there are no N- and C terminal charges, the capped peptides should bind better as they in this respect resemble the native protein more than uncapped peptides.

Preferred peptides according to the invention are:
Glu-Ala-Thr-Lys-Cys-Phe-Gln-Trp-Lys-Arg-Asn-Met-Arg-Lys-Val-Arg-Gly-Pro-Pro-Val-Ser-Cys-Ile-Lys-Arg (SEQ ID NO:2)
Glu-Ala-Thr-Lys-Cys-Phe-Gln-Trp-Lys-Arg-Ala-Met-Arg-Lys-Val-Arg-Gly-Pro-Pro-Val-Ser-Cys-Ile-Lys-Arg (SEQ ID NO:3)

In the description single-letter or three-letter symbols are used to denote the amino acids. These symbols, which are well known to man skilled in the art, have the following meaning: A = Ala = alanine, C = Cys = cysteine, D = Asp = aspartic acid, E = Glu = glutamic acid, F = Phe = phenylalanine, G= Gly = glycine, I- Ile = isoleucine, K = Lys = lysine, M= Met = methionine, N = Asn = asparagine, P = Pro = proline, Q = Gln = glutamine, R = Arg = arginine, S = Ser = serine, T = Thr = threonine, V = Val = valine, W = Trp = tryptophan, Orn = Ornithine, Nle = Norleucine and X = Xaa = a variable amino acid. Ac and NH₂ in some of the sequences denote an acetyl (CH₃CO-) group and an amino group, respectively, that have been used to modify the amino and the carboxy terminals of the peptides.

The advantage of the peptides according to the invention is that they comprise a modified version of the part of the human lactoferrin protein, which the inventors have found to be active with regards to the invention.

The peptides according to the invention are suitable for treatment and/or prevention of infections, inflammations, tumours, pain, wounds and scars. The term "treatment" used herein refers to curing, reversing, attenuating, alleviating, minimising, suppressing or halting the deleterious effects of a disease state, disease progression or other abnormal condition, and the term "prevention" used herein refers to minimising, reducing or suppressing the risk of developing a disease state or progression or other abnormal or deleterious conditions.

The infections treatable with the peptides or medicinal products according to the invention include infections caused by all kinds of pathogens, such as bacteria, viruses, fungi, etc.

It is also possible to treat different types of inflammations. Inflammation is a complex phenomenon marked i.a. by abnormal "redness" and swelling of tissues and organs, pain and heat in affected areas, capillary dilation, leucocyte infiltration, etc. Inflammation is primarily caused by exposure to bacterial and other noxious agents and physical injury. Inflammation has many forms and is mediated by a variety of different cytokines and other chemical signals. These mediators of inflammation include tumour necrosis factor-α (TNF-α), interleukin-1 (IL-1), interleukin-6 (IL-6), interleukin-8 (IL-8), and various colony-stimulating factors (CSFs).

As stated above, the peptides according to the invention are also suitable for treatment of tumours.

The peptides according to the invention may either be used as they are or be included in a medicinal product or a pharmaceutical preparation. The medicinal product or a pharmaceutical preparation according to the invention may also comprise substances used to facilitate the production of the pharmaceutical preparation or the administration of the preparations. Such substances are well known to people skilled in the art and may for example be pharmaceutically acceptable adjuvants, carriers and preservatives.

The peptides or medicinal products according to the invention can be administered to a patient either systemically or locally. The term "patient" used herein relates to any person at risk for or suffering from a disease state, disease progression or other abnormal or deleterious condition.

The systemic administration is suitable e. g. for treatment of urinary tract infection, colitis and tumours. The systemic administration can be undertaken by oral, nasal, intravenous, intraartery, intracavitary, intramuscular, subcutaneous, transdermal, suppositories (including rectal) or other routes known to those of skill in the art. Oral administration is preferred.

The local administration is suitable e. g. for treatment of skin infections, all infections and inflammations in mucosal membranes etc. The local administration can be undertaken by topical, oral, nasal, vaginal or oropharyngeal route. For treatment of local infections or inflammations in the skin or mucosal membranes the peptides or medicinal products according to the invention may e. g. be included in a gel, a cream, an ointment, or a paste.

In the method according to the invention an effective amount of a peptide according to the invention is administered to a patient. The term "effective amount" used herein relates to an amount sufficient to treat or prevent a disease state, disease progression or other abnormal or deleterious conditions.

The peptides or medicinal products and methods according to the invention are particularly well suited for treatment and/or prevention of urinary tract infection and colitis, but several other inflammatory and infectious diseases are also treatable according to the present invention, such as inflammatory bowel diseases, rheumatoid arthritis, conditions caused by the virus HIV-1, conditions caused by the virus CMV, and conditions caused by fungi, e.g. Candida species such as Candida albicans and Candida krusei, Aspergillus and Cryptococcus neoformans. This listing is in no way limiting the scope of the invention.

The peptides, medicinal products and methods according to the invention are also well suited for preventive medical care by reducing the risk of developing urinary tract infection or other inflammatory or infectious diseases in patients with an increased risk of attracting such complications. The peptides of the present invention are suited for are anti-inflammatory and immunomodulatory therapies, exemplified but not limited to:
1) Generally, treatment of inflammation and/or medical condition resulting from inflammation, and specifically,
2a) Intestine; Morbus Crohn, Colitis, Ulcerative colitis,
2b) Joints; Rheumatoid arthritis, Arthritis, Arthrosis, Localized disorders of muscles including muscle spasm, muscle tear, muscle injury, muscle strain, muscle sprain,
2c) Dermatology; Psoriasis, Eczema (excema), Dermatitis, Acne
2d) Heart; Pericarditis, Endocarditis Cardiac insufficiency,
2e) Pain; (further specified under 2f below).
2f) Nervous system; Alzheimer, Multiple Sclerosis, Carpal tunnel syndrome, Disc herniation, Cervical rhizopathy, Bells palsy, Acute spinal cord injury, Spinal cord compression, Spinal stenosis, Postherpetic neuralgia, Viral encephalitis, Viral meningitis, Menieres disease, Polio and postpolio complications, Chronic Inflammatory Demyelinating Polyneuropathy, Polyneuropathy, Trigminal neuralgia, Chronic epileptic disorders,
2g) Sensory organs; Glaucoma
2h) Mucosal surfaces (inflammation as a result of chemo/radiation therapy),
2i) Allergy,
2j) Autoimmune diseases

The peptides of the invention are further suited for prevention and treatment of wounds and scars in connection with conditions and procedure, exemplified but not limited to:
3a) surgical procedures on various tissues such as skin, muscles, tendons, nervous tissue, blood vessels, and at different locations of the body such as eyes, ears, vocal cord, hand, spinal cord, intra-abdominal cavity, intra-thoracic cavity, intra-cranial cavity, oral cavity, gynecological procedures, endometrios, phimosis,
3b) acne
3c) hypertrophic scars & keloids,
3d) pleuritis,
3e) peritoneal dialysis,

The peptides of the invention are further believed to have anti-angiogenetic effects and are therefore suited for treatment of :
4a) Cancer
4b) Rheumatoid arthritis

The peptides of the invention have anti-infectious effects, and are suited for the prevention and treatment of:
5a) Antibacterial effects: Upper and lower respiratory tract (tonsillitis, sinusitis etc.) Infections of the eye (e.g. conjunctivitis) Urinary tract infections Sexually transmitted diseases (including antimicrobial coating of condomes) Genital tract including vaginosis, vaginitis, cervicitis, endometritis, PID Gastrointestinal tract infections (systemic infections initiated in the GI) Central nervous system infections Infections of the skin (including staphylococci, for instance MRSA, nosocomial, wounds, burns), muscle, joints (e.g. septic arthritis), bone and hemopoietic system Infections related to the mouth, including parodontitis, gingivitis
5b) Antiviral effects: Upper and lower respiratory tract Sexually transmitted diseases Gastrointestinal tract infections (systemic infections initiated in the GI) Central nervous system infections
5c) Antifungal effects: Upper and lower respiratory tract (such as aphthae, mucocutanous candidiasis) Genitourinary tract, such as vulvovaginal candidiasis, balanitis, Gastrointestinal tract infections (systemic infections initiated in the GI) Central nervous system infections Infections of the skin (such as mucocutanous candidiasis)

The peptides, medicinal products and methods according to the invention may either be used alone, in combination with each other or in combination with conventional therapy.

According to the present invention it is also possible to include the peptides, in an effective amount, in any kind of food or beverage intended to reduce infections and/or inflammations in patients running an increased risk of such conditions due to an underlying disease, a low birth weight or a medical treatment. For example, it is possible to include the peptides, in an effective amount, in an infant formula food intended to inhibit harmful effects of bacteria, such as weight loss caused by inflammation induced by bacteria, viruses or fungi in infants. When the peptides according to the invention is to be used in food stuffs, e. g. for nutritional purposes, it is especially preferred to use peptides of natural origin. Since the peptides according to the invention have antimicrobial effects they can also be used as preservatives in different food stuffs and medicinal products such as gels, creams, ointments, pastes, solutions, emulsions etc.

The invention will now be further explained in the following examples. These examples are only intended to illustrate the invention and should in no way be considered to limit the scope of the invention.

Example. Microbicidal activity of a peptide of the invention The peptides were incubated with *E.coli, S.aureus* or *C.albicans* for 2h in BP. The peptides were diluted in two fold steps starting at 100 µg/ml or 200 µg/ml (*C.albicans*).

**Table 1.**

| | MMC99% | | |
|---|---|---|---|
| peptide | E.coli | S.aureus | C.albicans |
| HLBD1 | 50 | 50 | 200 |
| HLBD1Q24K | 12 | 25 | 50 |

HLBD1: E-A-T-K-C-F-Q-W-Q-R-N-M-R-K-V-R-G-P-P-V-S-C-I-K-R (SEQ ID NO:4)
HLBD1Q24K: E-A-T-K-C-F-Q-W-K-R-N-M-R-K-V-R-G-P-P-V-S-C-I-K-R (SEQ ID NO:2)

The microbicidal activity of the synthetic peptide HLBD1Q24K according to the present invention was considerably higher than the activity of the corresponding peptide derived from the sequence of native hLF.

## Claims

1. A peptide according to formula (I) R1-Cys-Phe-X1-X2-X3-X4-X5-X6-X7-Lys-Val-Arg-R2 Formula (I) wherein amino acid X1 is Gln or Ala, amino acid X2 is Trp or Leu, amino acid X3 is Gln, Ala, Orn, Nle or Lys, amino acid X4 is Arg, Ala or Lys, amino acid X5 is Asn, Ala, Orn or Nle, amino acid X6 is Met, Ala or Leu, amino acid X7 is Arg, Ala or Lys; with the proviso that simultaneous can not X1 be Gln, X2 be Trp, X3 be Gln, X4 be Arg, X5 be Asn, X6 be Met, and X7 be Arg;
R1 is either Lys or a peptide sequence selected from
| |
|---|
| Gly-Arg-Arg-Arg-Arg-Ser-Val-Gln-Trp-Cys-Ala-Val-Ser-Gln-Pro-Glu-Ala-Thr-Lys, |
| Arg-Arg-Arg-Arg-Ser-Val-Gln-Trp-Cys-Ala-Val-Ser-Gln-Pro-Glu-Ala-Thr-Lys, |
| Arg-Arg-Arg-Ser-Val-Gln-Trp-Cys-Ala-Val-Ser-Gln-Pro-Glu-Ala-Thr-Lys, |
| Arg-Arg-Ser-Val-Gln-Trp-Cys-Ala-Val-Ser-Gln-Pro-Glu-Ala-Thr-Lys, |
| Arg-Ser-Val-Gln-Trp-Cys-Ala-Val-Ser-Gln-Pro-Glu-Ala-Thr-Lys, |
| Ser-Val-Gln-Trp-Cys-Ala-Val-Ser-Gln-Pro-Glu-Ala-Thr-Lys, |
| Val-Gln-Trp-Cys-Ala-Val-Ser-Gln-Pro-Glu-Ala-Thr-Lys, |
| Gln-Trp-Cys-Ala-Val-Ser-Gln-Pro-Glu-Ala-Thr-Lys, |
| Trp-Cys-Ala-Val-Ser-Gln-Pro-Glu-Ala-Thr-Lys, |
| Cys-Ala-Val-Ser-Gln-Pro-Glu-Ala-Thr-Lys, |
| Ala-Val-Ser-Gln-Pro-Glu-Ala-Thr-Lys, |
| Val-Ser-Gln-Pro-Glu-Ala-Thr-Lys, |
| Ser-Gln-Pro-Glu-Ala-Thr-Lys, |
| Gln-Pro-Glu-Ala-Thr-Lys, |
| Pro-Glu-Ala-Thr-Lys, |
| Glu-Ala-Thr-Lys, |
| Ala-Thr-Lys, |
| Thr-Lys, |
R2 is either Gly or a peptide sequence selected from
Gly-Pro-Pro-Val-Ser-Cys-Ile-Lys-Arg,
Gly-Pro-Pro-Val-Ser-Cys-Ile-Lys,
Gly-Pro-Pro-Val-Ser-Cys-Ile,
Gly-Pro-Pro-Val-Ser-Cys,
Gly-Pro-Pro-Val-Ser,
Gly-Pro-Pro-Val,
Gly-Pro-Pro, and
Gly-Pro

2. A peptide according to claim 1 wherein R1 is Glu-Ala-Thr-Lys.

3. A peptide according to claim 1 or 2 wherein R2 is Gly-Pro-Pro-Val-Ser-Cys-Ile-Lys-Arg.

4. A peptide which is selected from Glu-Ala-Thr-Lys-Cys-Phe-Gln-Trp-Lys-Arg-Asn-Met-Arg-Lys-Val-Arg-Gly-Pro-Pro-Val-Ser-Cys-Ile-Lys-Arg (SEQ ID NO: 2); and Glu-Ala-Thr-Lys-Cys-Phe-Gln-Trp-Lys-Arg-Ala-Met-Arg-Lys-Val-Arg-Gly-Pro-Pro-Val-Ser-Cys-Ile-Lys-Arg (SEQ ID NO: 3).

5. A peptide according to any of claims 1 to 4, wherein the free COOH at the carboxy terminal end has been transformed into CONH₂.

6. A peptide according to any of claims 1 to 4, wherein the free the free NH₂ group at the amino terminal end has been transformed into the amide CH₃CONH.

7. A peptide according to any of claims 1 to 4, wherein the amino acid Cys, if present, has been replaced by an acetamidomethyl-cysteine.

8. A pharmaceutical composition comprising a peptide according to any of the claims 1 to 7.

9. A pharmaceutical composition according to claim 8 for treatment and/or prevention of infections, inflammations, tumours, pain, wounds and scars.

10. A pharmaceutical composition according to any one of the claims 8 to 9 formulated for oral administration, parenteral administration, or topical administration.

11. Food stuff comprising a peptide according to any of the claims 1 to 7.

12. Use of a peptide according to any one of the claims 1 to 7 for the production of a medicinal product for treatment and/or prevention of infections, inflammations, tumours, pain, wounds and scars.

13. Use according to claim 12, wherein the medicinal product is formulated for oral administration, parenteral administration, or topical administration.

14. A method for treatment or prevention of infections, inflammations, tumours, pain, wounds and scars wherein an effective amount of a peptide according to any of claims 1 to 7, fragments, and functionally equivalent homologues and analogues thereof, is administered to a patient.

15. A method according to claim 14, wherein the substance is orally, parenterally or topically administered.

16. A method according to claim 15, wherein the substance is included in a food stuff.

17. A method according to claim 16, wherein the substance is included in an infant formula food.

18. A peptide according to any one of the claims 1 to 7 for use as a medicament.

19. A peptide according to any one of the claims 1 to 7 for the treatment and/or prevention of infections, inflammations, tumours, pain, wounds and scars.

20. A peptide according to claim 19, formulated for oral administration, parenteral administration, or topical administration.
